(19) Europäisches Patentamt / European Patent Office / Office européen des brevets

(11) **EP 2 889 836 A2**

(12) **EUROPEAN PATENT APPLICATION**

(43) Date of publication:
**01.07.2015 Bulletin 2015/27**

(51) Int Cl.:
***G06T 7/00*** (2006.01)

(21) Application number: **14195616.9**

(22) Date of filing: **01.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **26.12.2013 JP 2013268216**

(71) Applicant: **Konica Minolta, Inc.**
**Tokyo 100-7015 (JP)**

(72) Inventor: **Matsumoto, Hiroaki**
**Tokyo, Tokyo 100-7015 (JP)**

(74) Representative: **Stanners, David Ralph**
**Urquhart-Dykes & Lord LLP**
**The Podium**
**1 Eversholt Street**
**London NW1 2DN (GB)**

(54) **Image processing apparatus and irradiating field recognition method**

(57) According to one implementation, an image processing apparatus includes the following. A setting unit sets a point in an irradiating field region of a radiation image as a reference point. A candidate point extracting unit extracts irradiating field edge candidate points based on the set reference point. A straight line candidate extracting unit extracts straight line candidates of the irradiating field edge based on the extracted irradiating field edge candidate points. A judging unit performs correct/incorrect judgment of the extracted straight line candidates. A recognizing unit recognizes the irradiating field region based on the straight line candidates judged to be correct. The judging unit calculates an evaluating value of the straight line candidates as the irradiating field edge. The judging unit performs correct/incorrect judgment of the straight line candidates based on the calculated evaluating value.

*FIG.4*

IRRADIATING FIELD RECOGNITION PROCESSING

SET POINT IN IRRADIATING FIELD REGION AS REFERENCE POINT — *S701*

EXTRACT IRRADIATING FIELD EDGE CANDIDATE POINT — *S702*

EXTRACT STRAIGHT LINE CANDIDATE OF IRRADIATING FIELD EDGE — *S703*

JUDGE CORRECT/INCORRECT OF EXTRACTED STRAIGHT LINE CANDIDATE AND REMOVE STRAIGHT LINE JUDGED TO BE INCORRECT — *S704*

DETERMINE IRRADIATING FIELD REGION — *S705*

END

**EP 2 889 836 A2**

**Description**

BACKGROUND

Field of the Invention

The present invention relates to an image processing apparatus and an irradiating field recognition method.

Description of Related Art

Conventionally, diagnosis is performed using a radiation image obtained by capturing a human body with radiation.

An irradiating field diaphragm which includes material through which radiation does not pass such as a lead plate is provided in a radiation generator. The purpose of the irradiating field diaphragm is to make the portion irradiated by radiation smaller so that the radiation is not irradiated in a portion not related to diagnosis or to prevent scattering radiation from the portion not related to the diagnosis entering into the portion necessary for diagnosis so that the resolution does not decrease. When the radiation image is obtained, typically capturing is performed so that the field that radiation is irradiated on the subject is limited.

Image processing of the radiation image is performed to obtain the radiation image suitable for diagnosis, etc. In such image processing, image processing conditions are determined from statistical characteristics of the radiation image (for example, maximum value, minimum value, average value, histogram, etc. of radiation image). Here, as described above, when capturing is performed limiting the radiation irradiating region using the irradiating field diaphragm, if the image processing condition is determined using image data within the irradiating field where the radiation is irradiated and the image data outside the irradiating field where the radiation is not irradiated, the entire radiation image is biased toward the small radiation amount by the image data outside the irradiating field. Then, suitable image processing is not performed on the image within the irradiating field region where diagnosis is necessary.

If the radiation image includes a region outside the irradiating field region, the following problems may occur, such as the portion becoming white and becoming too bright for diagnosis, the volume of the hard disk becoming large, or the like.

In view of the above, irradiating field recognition processing is performed to recognize the irradiating field region which is the region where radiation is irradiated and then the image processing is performed using the image within the irradiating field region, the blackening processing is performed to color the region outside the irradiating field region in black, and only the irradiating field region is cut out and stored in the hard disk, etc.

For example, as the irradiating field recognition processing, Japanese Patent Application Laid-Open Publication No. 2000-23952 describes the following technique. Image signals of the radiation image are used to detect a plurality of outline candidate points assumed to be on the outline of the irradiating field. When a predetermined number or more of the detected outline candidate points are on the same line, the line is detected as the irradiating field outline candidate line. Judgment is made of whether the detected irradiating field candidate line is correct or incorrect based on the distance from the center of the image, etc. The region surrounded by the irradiating field outline candidate line excluding the irradiating field outline candidate line judged to be incorrect in the correct/incorrect judgment is recognized as the irradiating field region.

In the CR (Computer Radiography) capturing apparatus used conventionally, the size of the cassette used in capturing is selected according to the size of the subject site. Therefore, the center of the image is included in the irradiating field and there has been no problem in judging whether the irradiating field outline candidate line is correct on the basis of the center of the image.

However, in capturing using FPD (Flat Panel Detector) which is spreading lately, since the FPD is expensive, a large size such as a half print size (14 inches x 17 inches) is prepared, and the image of a small irradiating field region is captured with a large size FPD. In this case, the center of the image is not always in the irradiating field region. With this, the correct/incorrect judgment of the irradiating outline candidate line may fail, and the irradiating field may not be recognized correctly.

SUMMARY

The present invention has been made in consideration of the above problems, and it is one of main objects to enhance accuracy of irradiating field recognition in a radiation image.

In order to achieve at least one of the above-described objects, according to an aspect of the present invention, there is provided an image processing apparatus including:

a setting unit which sets a point in an irradiating field region of a radiation image obtained by capturing a subject with radiation using an irradiating field diaphragm as a reference point;

a candidate point extracting unit which extracts a plurality of irradiating field edge candidate points which are candidates of points on an irradiating field edge in the radiation image based on the set reference point;

a straight line candidate extracting unit which extracts a plurality of straight line candidates of the irradiating field edge from the radiation image based on the extracted irradiating field edge candidate points;

a judging unit which performs correct/incorrect judgment of whether the extracted straight line candidates are correct or incorrect; and

a recognizing unit which recognizes the irradiating field region in the radiation image based on the straight line candidates judged to be correct by the judging unit,

wherein, the judging unit calculates an evaluating value of the straight line candidates as the irradiating field edge within a range where the straight line candidates are included in the irradiating field region or on the irradiating field edge, and performs correct/incorrect judgment of the straight line candidates based on the calculated evaluating value.

According to another aspect of the present invention, there is provided an irradiating field recognition method including:

setting a point in an irradiating field region of a radiation image obtained by capturing a subject with radiation using an irradiating field diaphragm as a reference point;

extracting an irradiating field edge candidate point which is a candidate of a point on an irradiating field edge in the radiation image based on the set reference point;

extracting a straight line candidate of the irradiating field edge from the radiation image based on the extracted irradiating field edge candidate point;

judging to perform correct/incorrect judgment of whether the extracted straight line candidate is correct or incorrect; and

recognizing the irradiating field region in the radiation image based on the straight line candidate judged to be correct, wherein, in the judging, an evaluating value of the straight line candidate as the irradiating field edge is calculated within a range where the straight line candidate is included in the irradiating field region or on the irradiating field edge, and correct/incorrect judgment of the straight line candidate is performed based on the calculated evaluating value.

According to the present invention, the accuracy of the irradiating field recognition in the radiation image can be enhanced.

BRIEF DESCRIPTION OF THE DRAWINGS

The present invention will become more fully understood from the detailed description given hereinbelow and the appended drawings, and thus are not intended to define the limits of the present invention, and wherein;

FIG. 1 is a diagram showing an example of an entire configuration of a radiation image capturing system of the present embodiment;

FIG. 2 is a block diagram showing a functional configuration of a capturing console shown in FIG. 1;

FIG. 3 is a flowchart showing capturing control processing performed by the control unit shown in FIG. 2;

FIG. 4 is a flowchart showing irradiating field recognition processing performed in step S7 shown in FIG. 3;

FIG. 5 is a diagram describing problems when a center of a radiation image is a reference point according to a conventional technique;

FIG. 6 is a diagram describing an example of an irradiating field edge candidate point being incorrectly extracted;

FIG. 7A is a diagram describing weighting edge strength E_w(i);

FIG. 7B is a diagram describing weighting edge strength E_w(i);

FIG. 8A is a diagram describing a line specified by r, $\theta$;

FIG. 8B is a diagram showing a curve representing the line passing through 5 edge candidate points on a r$\theta$ space (Hough space);

FIG. 9 is a diagram describing problems when a candidate of the line of the irradiating field edge is extracted according to the conventional technique;

FIG. 10A is a diagram showing Hough transform result before weighting in 90° direction;

FIG. 10B is a diagram showing Hough transform result after weighting in 90° direction;

FIG. 11A is a diagram describing correct/incorrect judgment of the candidate of the line of the irradiating field edge;

FIG. 11B is a diagram describing correct/incorrect judgment of the candidate of the line of the irradiating field edge; and FIG. 12 is a diagram describing an evaluating point of the region outside the irradiating field.

DETAILED DESCRIPTION OF PREFERRED EMBODIMENTS

An embodiment of the radiation image capturing system of the present invention is described in detail with reference to the drawings. However, the scope of the claims is not limited to the illustrated examples.

(Configuration of radiation image capturing system)

First, a configuration of a radiation image capturing system 100 of the present embodiment is described.

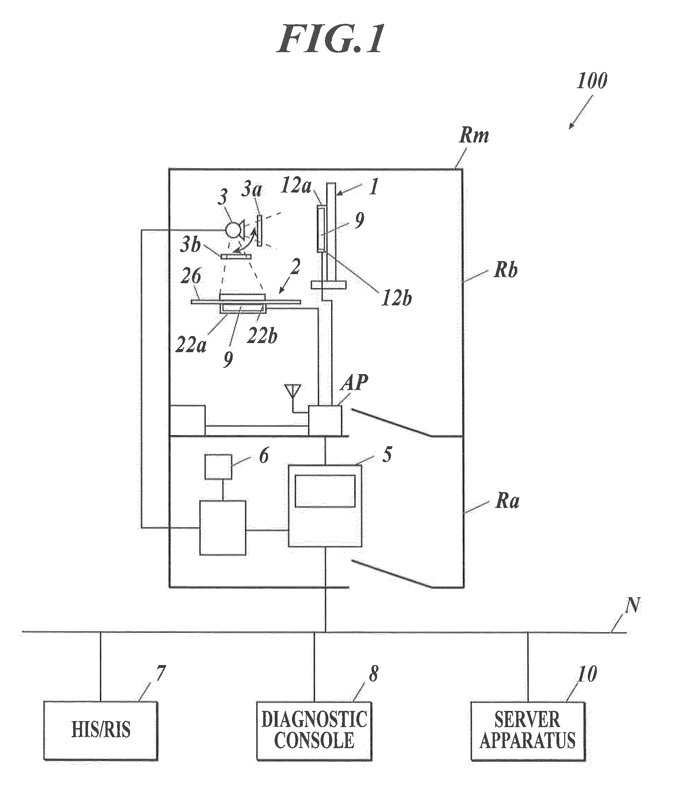

FIG. 1 is a diagram showing an example of an entire configuration of a radiation image capturing system 100 of the present embodiment. The radiation image capturing system 100 is a system in which radiation is irradiated on a diagnosis target site of a human body as a subject, and a static image of the subject is captured. FIG. 1 shows an example where the radiation image capturing system 100 is established in a capturing room Rm.

For example, the capturing room Rm includes, a bucky apparatus 1 for capturing in a standing position, a bucky apparatus 2 for capturing in a lying position, a radiation source 3, a capturing console 5, an operating table 6, and an access point AP. A front room Ra and a capturing performing room Rb are provided in the capturing room Rm. A capturing console 5 and an operating table 6 are provided in the front room Ra to prevent an operator such as a capturing technician from being irradiated.

Each apparatus of the capturing room Rm is described below.

The bucky apparatus 1 is an apparatus which holds the FPD (Flat Panel Detector) 9 in capturing in a standing position and performs capturing. The bucky apparatus 1 includes a holding unit 12a which holds the FPD 9 and a connector 12b to connect the connector of the FPD 9 attached to the holding unit 12a. The connector 12b transmits and receives data between the FPD 9 attached to the holding unit 12a and supplies electric power to the FPD 9. The bucky apparatus 1 includes an interface to transmit and receive data through a communication cable between an external device such as a capturing console 5, etc., through the access point AP and a foot switch which moves the holding unit 12a in a vertical direction or a horizontal direction.

The bucky apparatus 2 is an apparatus which holds the FPD 9 in capturing in a lying position and performs capturing. The bucky apparatus 2 includes a holding unit 22a which holds the FPD 9 and a connector 22b to connect the connector of the FPD 9 attached to the holding unit 22a. The connector 22b transmits and receives data between the FPD 9 attached to the holding unit 22a and supplies electric power to the FPD 9. The bucky apparatus 2 includes an interface to transmit and receive data through a communication cable between an external device such as a capturing console 5, etc., through the access point AP and a subject stage 26 where the subject is placed.

For example, the radiation source 3 is hung from the ceiling of the capturing room Rm. The radiation source 3 starts based on an instruction from the capturing console 5 when capturing is performed, and the radiation source 3 is adjusted to a predetermined position and a predetermined direction with the driving mechanism not shown. Then, the irradiating direction of the radiation is changed so that the radiation (X-ray) can be irradiated on the FPD 9 attached to the standing position bucky apparatus 1 and the lying position bucky apparatus 2. The radiation source 3 irradiates radiation once according to the radiation irradiating instruction from the operating table 6 and performs capturing of the static image.

In the radiation irradiating direction of the radiation source 3, irradiating field diaphragms 3a and 3b are provided, and the irradiating field of the radiation can be limited to a smallest region necessary for capturing the subject site according to operation by the user.

The capturing console 5 is an image processing apparatus which controls the radiation source 3 and the FPD 9 to control the capturing, and performs image processing on the radiation image generated by the capturing to transmit the image to the server apparatus 10. The capturing console 5 is connected to HIS/RIS (Hospital Information System/ Radiology Information System) 7, diagnostic console 8, server apparatus 10, etc. through a LAN (Local Area Network). The capturing console 5 controls the radiation source 3 and FPD 9 such as starting the above based on the capturing order information transmitted from the HIS/RIS 7 to perform capturing.

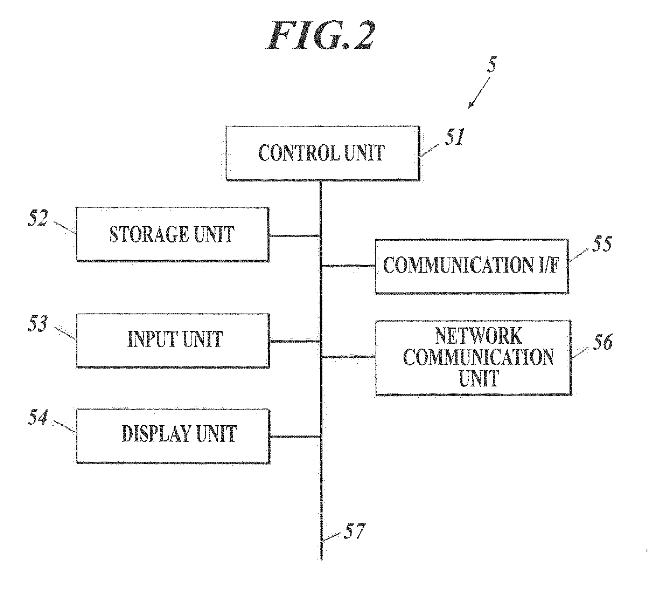

FIG. 2 shows an example of a configuration of a main section of the capturing console 5. As shown in FIG. 2, the capturing console 5 includes a control unit 51, a storage unit 52, an input unit 53, a display unit 54, a communication I/F 55, a network communication unit 56, and the like, and each unit is connected to each other with a bus 57.

The control unit 51 includes a CPU, a RAM, etc. The CPU of the control unit 51 reads various programs such as a system program, processing program, etc. stored in the storage unit 52 and expands the program in the RAM to perform various processing according to the expanded program.

For example, the control unit 51 make an inquiry to the HIS/RIS 7 through the network communication unit 56 each time a predetermined amount of time passes, and obtains the capturing order information newly registered in the HIS/RIS 7.

For example, the control unit 51 performs the later described capturing control processing and based on the capturing order information obtained from the HIS/RIS 7, the capturing console 5 controls the radiation source 3 and FPD 9 and performs capturing.

The storage unit 52 is composed of, for example, a HDD (Hard Disk Drive), a nonvolatile semiconductor memory, etc.

The storage unit 52 stores various programs and various pieces of data.

For example, in the storage unit 52, in addition to various programs to perform the capturing control processing, image processing parameters (a look-up table defining a gradation curve used in gradation processing, enhancement degree of frequency processing, etc.) to adjust the image data of the radiation image obtained by capturing to the image quality suitable for diagnosis for each site are stored.

Moreover, the capturing condition (radiation irradiating condition and image reading condition) is stored in the storage unit 52 corresponded to the capturing site (subject site). For example, the radiation irradiating condition includes the following, value of X-ray tube current, value of X-ray tube voltage, filter type, SID (Source to Image-receptor Distance), etc.

Moreover, the attribute information of the FPD 9 is stored in the storage unit 52. Here, the attribute information includes the attribute of the detecting element of the FPD 9 and the scintillator panel.

The capturing order information transmitted from the HIS/RIS 7 each time a predetermined amount of time passes is stored in the storage unit 52.

The input unit 53 includes a keyboard including a character input key, a numeral input key, various function keys, etc., and a pointing device such as a mouse, etc. The pressed signal of the key operated on the keyboard and the operating signal of the mouse are output to the control unit 51 as the input signal.

For example, the display unit 54 includes a monitor such as a CRT (Cathode Ray Tube), LCD (Liquid Crystal Display), etc., and various screens are displayed according to an instruction of a display signal input from the control unit 51.

A pressure sensitive type touch panel (not shown) where transparent electrodes are provided in a grid is formed on the screen of the display unit 54, and the display unit 54 and the input unit 53 can be a touch screen composed as one. In this case, in the touch panel, an XY coordinate of a pressure point pressed with the finger and the stylus pen are detected with a voltage value and the detected position signal is output to the control unit 51 as the operation signal. The display unit 54 can have a higher resolution than a monitor used in a typical PC (Personal Computer).

The communication I/F 55 is an interface which connects to the bucky apparatus 1, the bucky apparatus 2, the radiation source 3, and the FPD 9 through the access point AP and transmits and receives data wired or wirelessly. According to the present embodiment, the communication I/F 55 transmits a polling signal to the FPD 9 according to necessity through the access point AP.

The network communication unit 56 includes a network interface, etc., and transmits and receives data with the external device connected to the communication network N through the switching hub.

The operating table 6 is an input apparatus connected to a radiation source in the capturing room to input the radiation irradiated instruction.

The HIS/RIS 7 generates capturing order information according to registration operation by the operator based on a result of interviewing the patient. The capturing order information includes, for example, patient information such as name, sex, age, height, weight, etc. of the patient as the subject, information regarding the appointment of capturing such as the capturing site (subject site), the capturing direction, body position (standing position, lying position), the capturing method, etc. The capturing order information is not limited to the examples above, and other information can be included, or only some of the above described information can be included.

The diagnostic console 8 is a computer apparatus which obtains the radiation image from the server apparatus 10 and displays the obtained image so that the physician is able to interpret the image for diagnosis.

The FPD 9 is a radiation detector including a control unit, a detecting unit, a storage unit, a connector, a battery, a wireless communication unit and the like.

The detecting unit of the FPD 9 includes, for example, a glass substrate, etc. A plurality of detecting elements are arranged two dimensionally in a predetermined position on the substrate. The detecting element detects the radiation which is irradiated from the radiation source 3 and which at least passes through the subject according to the intensity and the detected radiation is converted to an electric signal to be accumulated. The detecting element includes a semiconductor image sensor such as a photodiode. The detecting elements are connected to a switching unit such as a TFT (Thin Film Transistor), etc., and the accumulating and reading of the electric signal is controlled by the switching unit.

The control unit of the FPD 9 controls the switching unit of the detecting unit based on the image reading condition input from the capturing console, switches the reading of the electric signal accumulated in each detecting element, and reads the electric signal accumulated in the detecting unit to generate the image data of the radiation image (static image). Then, the control unit outputs the generated image data to the capturing console 5 through the connector and the bucky apparatus 1 or 2. The pixels composing the generated static image show a signal value output from each of the detecting element of the detecting unit. The signal value becomes larger as the radiation intensity reaching the detecting elements becomes higher. As the signal value becomes larger, the pixel is drawn blacker (with higher density).

The connector of the FPD 9 is connected to the connector of the bucky apparatuses 1 and 2, and data is transmitted and received between the bucky apparatus 1 or 2. The connector of the FPD 9 supplies electric power supplied from the connector of the bucky apparatus 1 or 2 to the functional units. Alternatively, a battery can be charged.

When the FPD 9 is not attached to the bucky and is used alone, the FPD 9 is battery driven and communicates wirelessly. When the FPD 9 is attached to the bucky apparatus 1 or 2, it is possible to switch from battery/wireless method to wired/ electric power supply method by connector connection. Therefore, there is no need to worry about the battery running out even when the static image of a plurality of patients are captured successively.

The server apparatus 10 includes a database storing the image data of the radiation image transmitted from the capturing console 5 and the interpretation report corresponded with the capturing order information. The server apparatus 10 reads the radiation image from the database according to a request from the diagnostic console 8 and transmits the radiation image to the diagnostic console 8.

(Operation of Radiation Image Capturing System)

Next, the operation of the radiation image capturing system 100 is described.

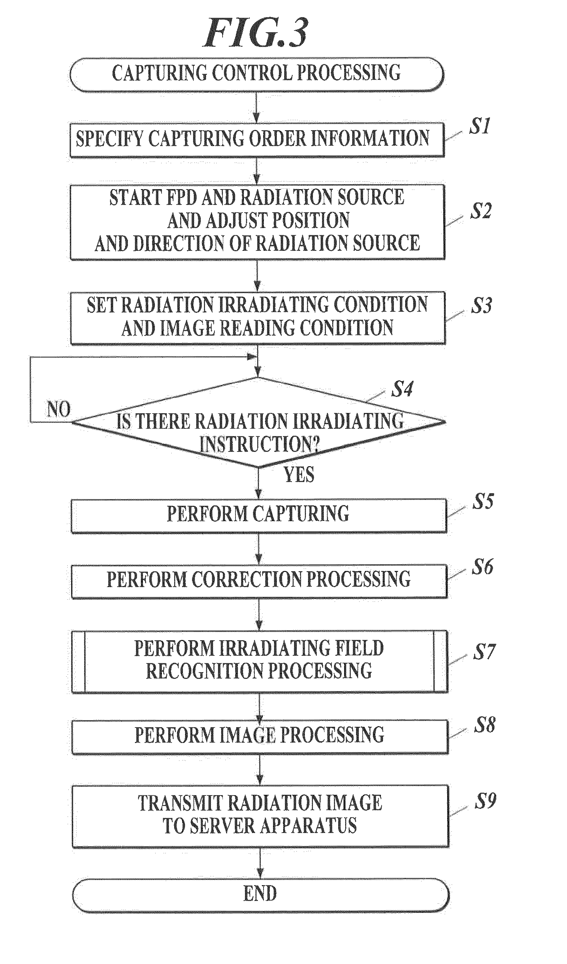

FIG. 3 shows a flow of the capturing control processing performed in the capturing console 5. The capturing control processing shown in FIG. 3 is performed by the control unit 51 of the capturing console 5 according to an instruction from the input unit 53 in coordination with the program stored in the storage unit 52.

First, the operator such as the capturing technician sets the FPD 9 in the bucky apparatus 1 or the bucky apparatus 2. Then, the operator operates the input unit 53 of the capturing console 5 to display the capturing order list screen displaying a list of the capturing order information on the display unit 54. Then, the input unit 53 is operated to specify the capturing order information of the capturing target from the capturing order list screen and the bucky ID to be used in the capturing.

In the capturing console 5, when the capturing order information of the capturing target and the bucky ID are specified with the input unit 53 (step S1), the control unit 51 starts the radiation source 3 and the FPD 9 and adjusts the direction and the positon of the radiation source 3 according to the used bucky apparatus. When the subject is positioned and the capturing technician adjusts the position, etc. of the FPD 9 and the bucky apparatus suitable for the subject, the control unit 51 adjusts the direction and the position of the radiation source 3 according to the above (step S2). The control unit 51 reads from the storage unit 52 the radiation irradiating condition and the image reading condition according to the site to be captured, sets the radiation irradiating condition in the radiation source 3, and sets the image reading condition in the FPD 9 through the bucky apparatus (step S3).

When the irradiating field diaphragm 3a or 3b is adjusted according to the direction of the radiation source 3 and the capturing preparation is complete, the capturing technician operates the operating table 6 to input the radiation irradiating instruction.

When the radiation irradiating instruction is input from the operating table 6 (step S4; YES), the control unit 51 controls the radiation source 3 and the FPD 9 used in capturing and captures the subject (step S5). Specifically, the radiation image which is a static image of the subject and one or a plurality of dark images for offset correction are captured under the condition set in step S3. The image data of the radiation image of the subject and the dark image generated by capturing is input to the capturing console 5 through the bucky apparatus from the FPD 9 and stored in the storage unit 52.

Next, the control unit 51 performs correction processing on the radiation image obtained by capturing (step S6) and advances the processing to step S7. In step S6, the control unit 51 performs correction processing such as offset correction processing using the above described dark image, gain correction processing, defective pixel correction processing, lag (afterimage) correction processing, etc. according to necessity based on the attribute information of the FPD 9 stored in the storage unit 52.

When there are a plurality of FPD 9, the attribute information is stored in the storage unit 52 corresponded with the identification ID of each FPD 9, the identification ID is obtained from the FPD 9 through the bucky apparatus used, and the correction processing and the later described image processing are performed based on the attribute information corresponding to the obtained identification ID.

Next, the control unit 51 performs irradiating field recognition processing on the corrected radiation image to recognize the range of the irradiating field in the radiation image (step S7).

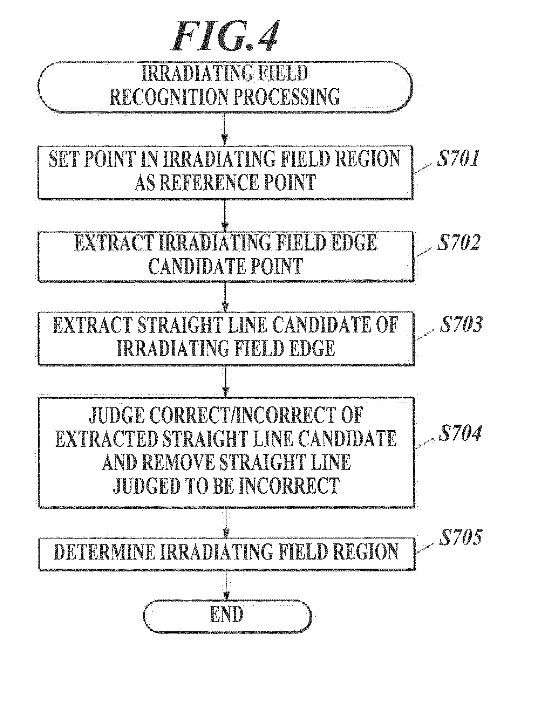

FIG. 4 shows a flow of the irradiating field recognition processing performed in step S7. The irradiating field recognition processing of FIG. 4 is performed by the control unit 51 of the capturing console 5 in coordination with the program stored in the storage unit 52.

First, the control unit 51 sets the reference point in the irradiating field region of the radiation image (step S701).

Then, for example, Japanese Patent No. 3923131 proposes a method which sets the center of the radiation image as the reference point to perform irradiating region recognition, sets a plurality of straight lines radially from the center of the image toward the edge of the radiation image, obtains the edge candidate point considered to be the outline of the irradiating field on the straight lines, and recognizes the irradiating field region based on the edge candidate points. In conventional CR capturing, the size of the cassette (photostimulable phosphor plate) used in capturing is selected according to the size of the subject site. Therefore, the center of the image is included in the irradiating field region and there is no problem in setting the center of the image as the reference point.

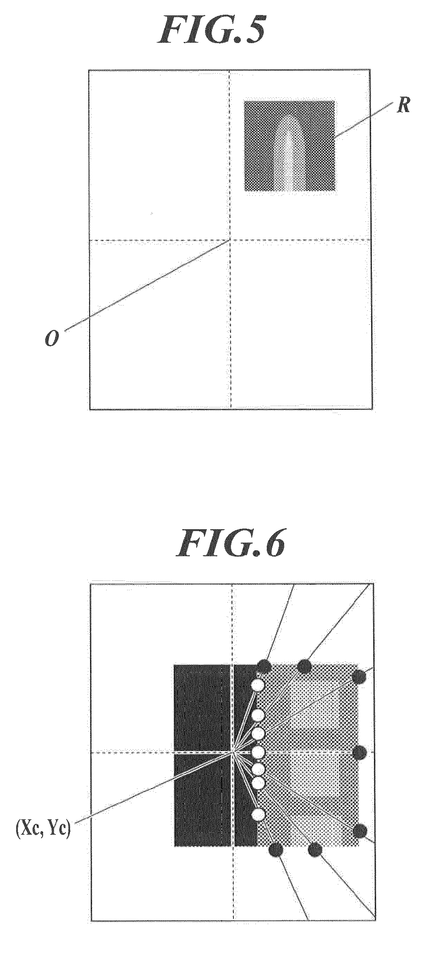

However, in capturing using FPD which is spreading lately, since the FPD is expensive, for example, FPD in a large size such as a half print is prepared, and capturing of the image with a small irradiating field is performed with such large sized FPD. In this case, as shown in FIG. 5, the center point O may not be in the irradiating field region R, and if the irradiating field recognition processing is performed using the center point O as reference, the recognition of the irradiating field region fails.

Moreover, for example, Japanese Patent No. 4280729 describes that the region outside the irradiating field in the radiation image is a region where the radiation is shielded, and the pixel value (signal value) tends to be smaller than inside the irradiating field. Therefore, the centroid of the pixel with the pixel value equal to or higher than the predetermined threshold value is set as the reference point in the irradiating field region. However, since there are pixels with a large signal value due to the ghost image in the surroundings of the region outside the irradiating field, according to the above method, the reference point may be calculated in a region outside the irradiating field.

Step S701 focuses on the following features, (1) the signal value is high because the radiation is irradiated in the irradiating field region, (2) there are a large number of edges because the subject (structure) is seen in the irradiating field region. The reference point which is the start of the above described radial straight line is set based on the edge strength and the signal value of the radiation image. Therefore, wherever the irradiating field region is in the image, or even if the radiation amount is high outside the irradiating field, the reference point can be accurately set in the irradiating field region.

In step S701, the control unit 51 sets the reference point according to the following processing of (a1) to (a4).

(a1) First, the control unit 51 generates a difference image in the vertical direction of the radiation image and the difference image in the horizontal direction.

For example, when the horizontal coordinate of the radiation image is i and the vertical coordinate of the radiation image is j, and the signal value of the pixel with the coordinates (i, j), is f (i, j), the difference value g1 (i, j) with the same coordinates (i, j) in the difference image in the vertical direction can be obtained by the (formula 1) below, and the difference value g2 (i, j) with the same coordinates (i, j) in the difference image in the horizontal direction can be obtained by the (formula 2) below.

$$g1\ (i,\ j) = f\ (i,\ j{+}1) - f\ (i,\ j{-}1)\ ...\ (formula\ 1)$$

$$g2\ (i,\ j) = f\ (i{-}1,\ j) - f\ (i{+}1,\ j)\ ...\ (formula\ 2)$$

The difference values g1 (i, j) and g2 (i, j) can be a difference between signal values of adjacent pixels. A large difference value of the signal value shows that there is a strong edge.

(a2) Next, the control unit 51 generates one edge strength image from the difference image in the vertical direction and the difference image in the horizontal direction. Specifically, as shown in (formula 3), in each pixel, the vector of the strength of the edge in the horizontal direction (in other words, the value of the difference image in the horizontal direction) and the vector of the strength of the edge in the vertical direction (in other words, the value of the difference image in the vertical direction) are added, and the size of the added vector is calculated as the edge strength E (i, j) of the pixel.

$$E\ (i,\ j) = \sqrt{\{g1\ (i,\ j)^2 + g2\ (i,\ j)^2\}}\ ...\ (formula\ 3)$$

(a3) Next, the control unit 51 generates the weighting edge strength image in which the edge strength of each pixel in the edge strength image is weighted with the signal value. As described above, the radiation is irradiated in the irradiating field region, and therefore the signal value becomes high. The edge strength E (i, j) is weighted higher as the signal becomes higher and is weighted lower as the signal becomes lower. With this, the weighting edge strength E_w (i, j) is calculated for the coordinates (i, j) in the weighting edge strength image. Specifically, E_w (i, j) can be obtained by the following (formula 4).

$$E\_w\ (i,\ j) = E\ (i,\ j) \times (I\ (i,\ j)/C) \ ... \ \text{(formula 4)}$$

Here, I (i, j) represents the signal value and C represents normalization constant (here, maximum signal value).

(a4) Next, the control unit 51 obtains a centroid position (Xc, Yc) in the weighting edge strength image with the (formula 5) below, and this is set as the reference point.

X represents the number of pixels in the horizontal direction of the weighting edge strength image, and Y represents the number of pixels in the vertical direction of the weighting edge strength image.

(formula 5)

$$X_c = \frac{\sum_{i=1}^{X} \sum_{j=1}^{Y} E\_w\ (i,\ j) \times i}{\sum_{i=1}^{X} \sum_{j=1}^{Y} E\_w\ (i,\ j)} \qquad Y_c = \frac{\sum_{i=1}^{X} \sum_{j=1}^{Y} E\_w\ (i,\ j) \times j}{\sum_{i=1}^{X} \sum_{j=1}^{Y} E\_w\ (i,\ j)}$$

As described above, in step S701, the centroid position of the weighting edge strength image where the weighting of the region outside the irradiating field with the low signal is low is set as the reference point (Xc, Yc), and therefore, the reference point (Xc, Yc) can be set within the irradiating field region.

In the processing afterwards, the signal value of each pixel is obtained from the radiation image, and the edge strength is obtained from the above described edge strength image. Moreover, the direction vector of the edge of each pixel is obtained from the difference image in the vertical direction and the difference image in the horizontal direction.

When the reference point is set, the control unit 51 extracts the irradiating field edge candidate point (candidate of the point on the irradiating field edge) (step S702).

Here, as the method of obtaining the irradiating field edge candidate point (hereinafter referred to as candidate point) for example, Japanese Patent No. 3923131 proposes setting a plurality of radial straight lines from the reference point (center of image) to the image edge, and setting the point with the largest difference value between adjacent pixels on the straight line as the candidate point.

However, as described in FIG. 6, when the irradiating edge is composed of the human body and the region outside the irradiating field (locations with black points in FIG. 6), the difference value between pixels may become small. Further, when there is a location where the human body is adjacent to the region where the radiation directly reaches the FPD 9 (pass-through region) (location with white points in FIG. 6) on the straight line, since the difference value of the signal value between pixels is large, the candidate point may be obtained in the boundary between the human body and the pass-through region, and the candidate point may not be obtained on the irradiating field outline.

In step S702, not only the edge strength but also the signal value is used to extract the candidate point, and with this, the accuracy of extracting the candidate point is enhanced. The process focuses on the feature that when there is a candidate point in the irradiating field edge, the outside is the region outside the irradiating field, and therefore, the radiation amount is low (in other words, the signal value is low). For each of the points on the straight line drawn radially (drawn in each predetermined angle) from the reference point (Xc, Yc) to the image edge, the edge strength is weighted based on the signal value of the outside, and the point with the maximum value of the weighting edge strength is to be the candidate point.

Specifically, in step S702, the control unit 51 performs the following processing of (b1) to (b3).

(b1) First, the control unit 51 obtains the weighting coefficient W(i) of the point in a distance i on each straight line drawn from the reference point (Xc, Yc) to the image edge with the following (formula 6).

$$W(i) = 1.0 - M\_c\ (i)\ /\ M\_p \ ... \ \text{(formula 6)}$$

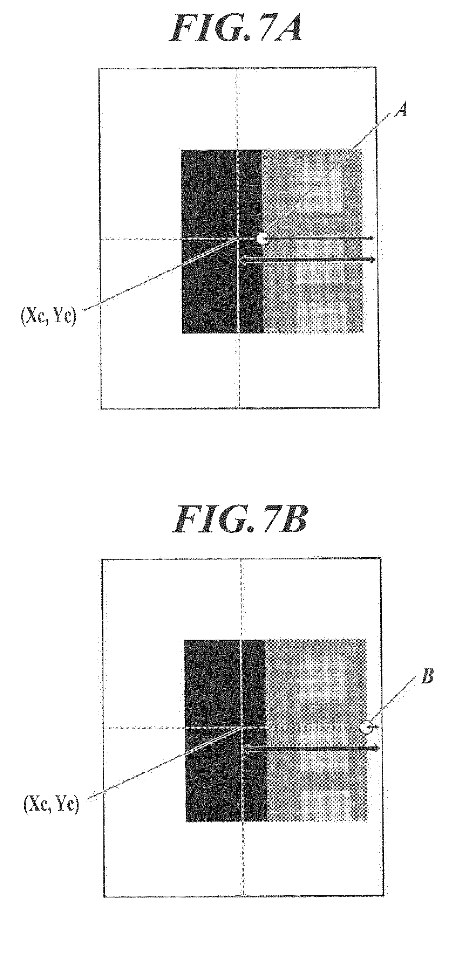

Here, M_c (i) represents the maximum value of the signal value in the interval shown with a thin arrow in FIG. 7A and FIG. 7B, in other words, the maximum value of the signal value from the coordinates of the point on the straight line, drawn from the reference point (Xc, Yc) to the image edge, to the image edge. M_p represents the maximum value of the signal value in the interval shown with a thick arrow in FIG. 7A and FIG. 7B, in other words, the maximum value of the signal value of the interval from the reference point (Xc, Yc) to the edge of the image.

(b2) Next, the control unit 51 calculates the weighting edge strength E_w (i) of the point in the distance i on each straight line drawn from the reference point (Xc, Yc) to the image edge with the following (formula 7).

$$E\_w\ (i) = E\ (i) \times W\ (i)\ ... \ (formula\ 7)$$

E (i) represents the value of the pixel of the edge strength image (edge strength).

The above straight line is drawn radially for each predetermined angle from the reference point (Xc, Yc) to the image edge, and the above described E_w (i) is calculated for each point on each straight line.

(b3) Then, the control unit 51 extracts the point with the largest E_w (i) on each straight line as the irradiating field edge candidate point.

The point A shown in FIG. 7A has a large edge strength E (i) since the point is on the boundary between the pass-through region and the human body. However, the signal value of the outside of the point A becomes high because the point A is within the irradiating field, and the weighting coefficient W (i) becomes small according to the above (formula 6). Therefore, the E_w (i) becomes small.

Turning to point B shown in FIG. 7B, the point B is in the boundary between the human body and the region outside the irradiating field. Therefore, the edge strength E (i) is approximately the middle. However, the weighting coefficient W (i) becomes large according to the above (formula 6) because the outside of the point B is the region outside the irradiating field. Therefore, the E_w (i) of point B becomes higher than the E_w (i) of point A, and the point B is extracted as the candidate point of the irradiating field edge.

As described above, in step S702, the edge strength is corrected according to the signal value showing the magnitude of the radiation amount outside, and therefore, the candidate point can be extracted in the irradiating field edge with a weak edge at the boundary between the human body.

Next, the control unit 51 extracts the straight line candidate of the irradiating field edge (step S703).

Here, as the method of obtaining a straight line of the irradiating field edge candidate, for example, Japanese Patent No. 3923131 describes the following technique. A plurality of radial straight lines are set from the reference point to the image edge and the largest difference value between adjacent pixels on the straight line is set as the edge candidate point (corresponding to the above described irradiating field edge candidate point). Hough transform is applied to the edge candidate point to obtain a predetermined number of reference candidate lines and such reference candidate lines are determined to be the straight line of the irradiating field edge.

Here, Hough transform is described.

There are a countless number of straight lines which pass a point $(x_0, y_0)$ on the xy coordinate system, and the formula of the straight line which pass $(x_0, y_0)$ can be represented by the following (formula 8).

$$r\ (\theta) = x_0 \cos\theta + y_0 \sin\theta\ ...\ (formula\ 8)$$

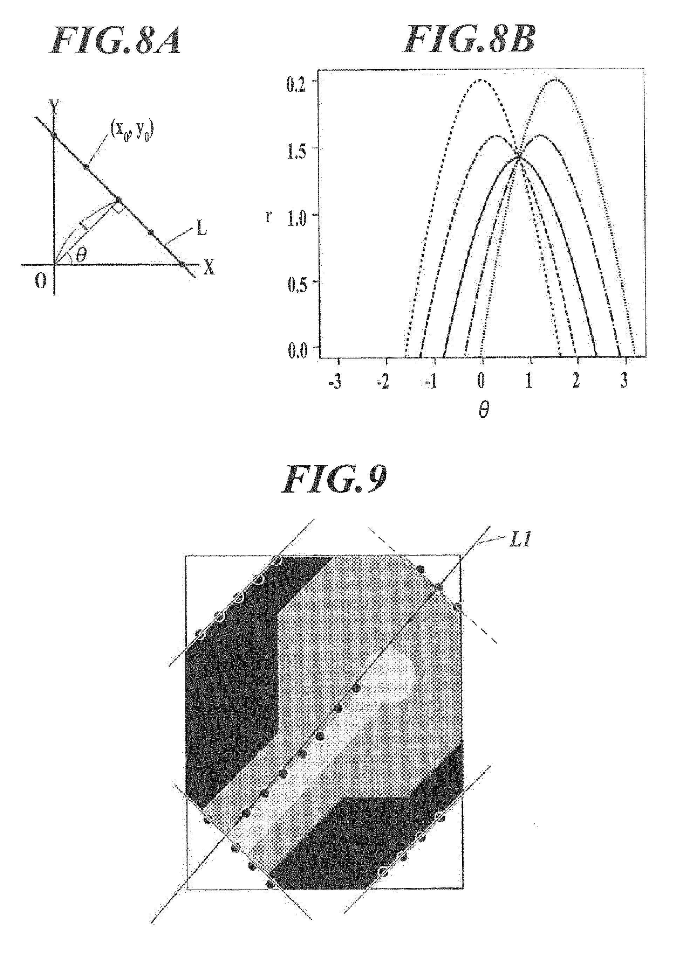

Here, as shown in FIG. 8A, r represents the length of the perpendicular from the point of origin O of the xy coordinate system toward the straight line L passing through coordinates $(x_0, y_0)$, and θ represents the angle between this perpendicular and the x-axis. Each line L which passes through the coordinates $(x_0, y_0)$ is a line in which r and θ are changed, and in rθ space (Hough space) the group of lines L is represented by one of the curves shown in FIG. 8B.

For example, when the curve showing the straight line which passes the edge candidate points (for example, 5 points) is to be obtained, 5 curves are obtained in the rθ space as shown in FIG. 8B. The straight line specified by the r, θ of the intersection where the plurality of curves among the 5 curves intersect is the straight line which passes the edge candidate point in the number of curves intersecting in the intersection. Japanese Patent No. 3923131 describes counting (polling) the number of curves intersecting in each intersection, extracting the intersection position in order from the larger counting value, obtaining the straight line in the xy coordinate system representing the extracted intersection position, and extracting the obtained straight line as the reference candidate line. The polling for one edge candidate point is within the range of 180°.

However, the number of curves that overlap on the intersection represent the number of edge candidate points on the straight line specified by the intersection. As shown in FIG. 9, when the irradiating field edge is in a region close to the image edge, the number of edge candidate points on the straight line of the irradiating field edge becomes small. For example, the number of edge candidate points on the straight line shown with a dotted line in the upper right of FIG. 9 is 3, and the number is very small. Therefore, the counting value may become small and the line may not be extracted as the reference candidate line (corresponding to the straight line candidate of the irradiating field edge). Moreover, as shown in FIG. 9, when the edge candidate point is obtained on the human body by mistake, a few points may coincidentally align in a straight line (for example, L1 of FIG. 9). In such case, the counting value of the irradiating field edge in the image edge becomes small, and the counting value corresponding to the straight line connecting the false edge candidate points on the human body becomes larger. Therefore, the reference candidate line may not be obtained in the irradiating field edge or the reference candidate line may be obtained on the human body.

In step S703, weighting is performed depending on how much each straight line passing through each irradiating field edge candidate point is like the irradiating field edge, and polling of the Hough transform is performed for each straight line passing each irradiating field edge candidate point. Therefore, the above problems are solved. Here, examples of the features of a straight line like the irradiating field edge include the following (1) the edge is strong and the outside is to be outside the irradiating field and therefore, the radiation amount is low (in other words, the signal value is low), (2) the possibility that the line is orthogonal to the edge direction in the irradiating field edge candidate point becomes high, (3) the distance from the reference point (Xc, Yc) is far, and the like.

Specifically, in step S703, the control unit 51 performs the following processing of (c1) to (c4).

(c1) First, a number i is set for the irradiating field edge candidate point, and the control unit 51 obtains the length r of the perpendicular from the reference point (Xc, Yc) and the angle $\theta$ between the perpendicular and the x-axis for each straight line passing through i, and obtains the value $Hp\_i (r, \theta)$ polling in the coordinate in the $r\theta$ space corresponding to each straight line with the following (formula 9).

$$Hp\_i (r, \theta) = E\_W\_i \times W\_Ip\_i (\theta) \times W\_D\_i \ldots \text{(formula 9)}$$

Here, $E\_W\_i$ represents the weighting edge strength, $W\_Ip\_i (\theta)$ represents the edge direction weighting coefficient, $W\_D\_i$ represents the distance weighting coefficient, and the above corresponds to the above described features (1) to (3) respectively.

The weighting edge strength $E\_W\_i$ is the weighting edge strength for the irradiating field edge candidate point i obtained in step S702, and therefore, the edge direction weighting coefficient and the distance weighting coefficient are described below.

First, the edge direction weighting coefficient $W\_Ip\_i (\theta)$ is described.

As described above, when a straight line passing through the irradiating field edge candidate point i is a straight line of the irradiating field edge, there is a high possibility that the straight line is perpendicular to the direction vector of the edge of the irradiating field edge candidate point i. In other words, there is a high possibility that the direction vector of the edge of the irradiating field edge candidate point i and the normal vector of the straight line are in the same direction. Therefore, the straight line with a normal vector in the same direction as the direction vector of the edge in the irradiating field edge candidate point i is weighted highly with the edge direction weighting coefficient $W\_Ip\_i (\theta)$.

Specifically, $W\_Ip\_i (\theta)$ ($W\_Ip\_i (\theta) > 0$) is an inner product of the normal vector of the straight line passing through the irradiating field edge candidate point i (normal vector of the straight line represented by the above (formula 8)) and the direction vector of the edge of the irradiating field edge candidate point i, and the above is represented by the following (formula 10).

$$W\_Ip\_i (\theta) = \cos\theta \cdot dX + \sin\theta \cdot dY \ldots \text{(formula 10)}$$

Here, the normal vector of the straight line passing through the irradiating field edge candidate point i is represented by the following.

$$(\cos\theta, \sin\theta) \ldots \text{(formula 11)}$$

Moreover, dX is the vector of the edge of the irradiating field edge candidate point i in the x direction, dY is the vector of the edge of the irradiating field edge candidate point i in the y direction. The dX can be obtained from the difference image in the horizontal direction generated in step S701, and dY can be obtained from the difference image in the vertical direction generated in step S701. When the vectors are in the same direction the inner product is 1, when the vectors are orthogonal the inner product is 0, and when the vectors are in the opposite direction the inner product is -1.

In other words, the straight line in the direction perpendicular to the edge of the irradiating field edge candidate point i can be weighted strongly with the W_Ip_i ($\theta$). It is possible to not pick up the straight line in another direction just because the edge strength is high.

Next, the distance weighting coefficient W_D_i is described.

The W_D_i is the coefficient represented by the following (formula 12).

$$W\_D\_i = (D/Dmax) \ ... \ (formula \ 12)$$

Here, D is a distance from the reference point (Xc, Yc) to the irradiating field edge candidate point i, and Dmax is the length of the image diagonal line.

The control unit 51 obtains the above described Hp_i (r, $\theta$) of the coordinate in the r$\theta$ space representing each straight line passing through the candidate point for all of the irradiating field edge candidate points i.

The polling value Hp_i (r, $\theta$) is a value multiplying the 3 weighting coefficients of E_W_i, W_Ip_i($\theta$), W_D_i. Weighting with a higher accuracy than the conventional method can be performed even by 1 or a combination of 2 of the above. Moreover, either of the edge strength which is the component of the weighting edge strength E_W_i or the signal value of the radiation image between the point where the straight line passing the irradiating field edge candidate point i and the reference point (Xc, Yc) intersects with the image edge and the irradiating field edge candidate point i, or a combination of either of the above and the W_Ip_i ($\theta$), W_D_i can be the polling value Hp_i (r, $\theta$).

(c2) Next, the control unit 51 calculates the H (r, $\theta$) obtained by the following (formula 13) as the final Hough transform result.

$$H \ (r, \theta) = sum \ (Hp\_i \ (r, \theta)) \ ... \ (formula \ 13)$$

In other words, the polling value added in each point in the r$\theta$ space is obtained as the final result of the Hough transform.

For example, according to the process above, even if the straight line has only 3 irradiating field edge candidate points, if there are features like the irradiating field edge, the total of the polling value becomes large, and the straight line can be extracted as the straight line candidate of the irradiating field edge. Moreover, as for the straight line without the features (1) to (3) of the irradiating field edge such as the straight line of the human body, even if there are many irradiating field edge candidate points, it is possible to perform processing so that such line is not extracted as the straight line candidate of the irradiating field edge.

(c3) Here, the shape of the irradiating field does not have to be a rectangle, however, the shape is mostly a rectangle. The control unit 51 performs weighing in the 90°C direction on the obtained Hough transform result so that the straight line of the irradiating field edge can be surely extracted.

Specifically, the control unit 51 performs the following (c3-1) to (c3-3) for each $\theta$ in the r$\theta$ space.

(c3-1) The maximum value M1 ($\theta$) of the polling value is obtained.

(c3-2) Next, M2 ($\theta$) with the strength of M1 ($\theta$) weakened is calculated with the following (formula 14).

$$M2 \ (\theta) = M1 \ (\theta) \wedge (1/4) \ ... \ (formula \ 14)$$

Here, 1/4 is a coefficient to weaken the strength and the value is not limited to this.

(c3-3) Next, the weighted M ($\theta$) is added in the direction of 90°, 180°, 270° from $\theta$

Here,

$$M \ (\theta) = M2 \ (\theta) \times Wt \ ... \ (formula \ 15)$$

Wt is a predetermined coefficient.

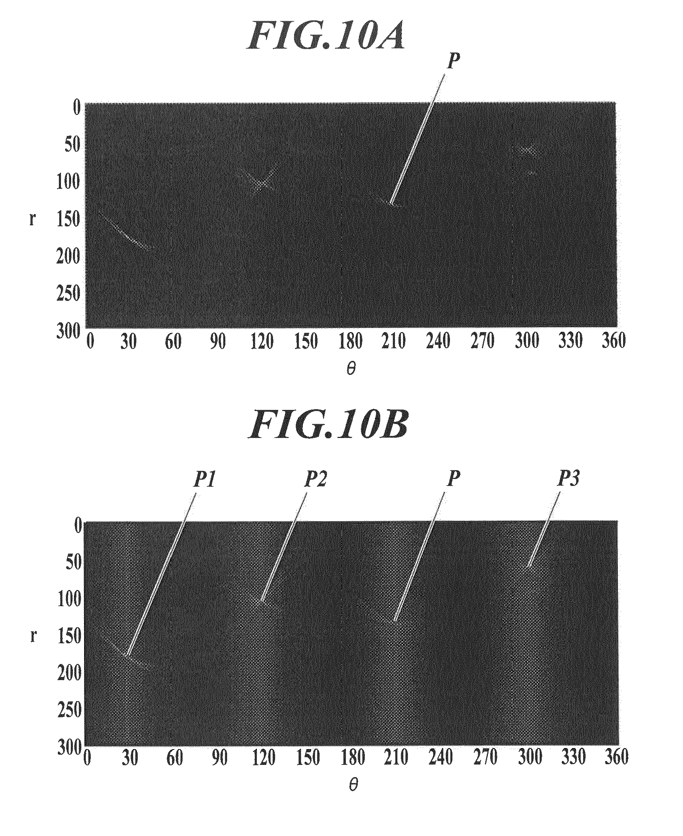

FIG. 10A shows a Hough transform result before weighting in the 90° direction. FIG. 10B shows the Hough transform result weighting in the 90° direction. In the graph shown in FIG. 10A and FIG. 10B, the coordinate with the larger value is shown whiter.

For example, the point P in FIG. 10A corresponds to a straight line (straight line P) in the original image. Since the length of the straight line P is short, the number of candidate points on the straight line P is small, and the polling value of the point P is small. However, when the weighting in the 90° direction is performed, since P is in the 90° direction of the points P1, P2, and P3 which have high polling values, as shown in FIG. 10B, a relatively large value M (θ) is added to P and the point P can be easily obtained as the straight line candidate.

(c4) After weighting in the 90° direction, the control unit 51 extracts the straight line corresponding to the predetermined number of points in order from the large polling value in the coordinate on the rθ space showing the weighting Hough transform as the straight line candidate of the irradiating field edge.

Returning to FIG. 4, after the straight line candidate of the irradiating field edge is obtained, the control unit 51 judges whether the obtained straight line candidate is correct or incorrect. The straight line which is judged to be incorrect and which is therefore not the irradiating field edge is removed (step S704).

Here, as described above, Japanese Patent Application Laid-Open Publication No. 2000-23952 proposes the following method. Judgment is made whether the candidate line is correct or incorrect based on the distance from the center of the image. After removing the irradiating field outline candidate line detected by mistake, the irradiating field outline candidate line is to be the irradiating field outline. However, as described above, in capturing using FPD being performed lately, the center point is not always in the irradiating field region, and in this case, judgment of whether the candidate line is correct or incorrect may fail. Moreover, when the correct or incorrect judgment of the candidate line fails, the irradiating field cannot be suitably recognized. Therefore, there is a demand for a highly accurate judgment of whether the line is correct or incorrect.

In view of the above, in step S704, the following processing of (d1) to (d4) is performed to judge whether the straight line candidate is correct or incorrect and to remove the incorrect straight line.

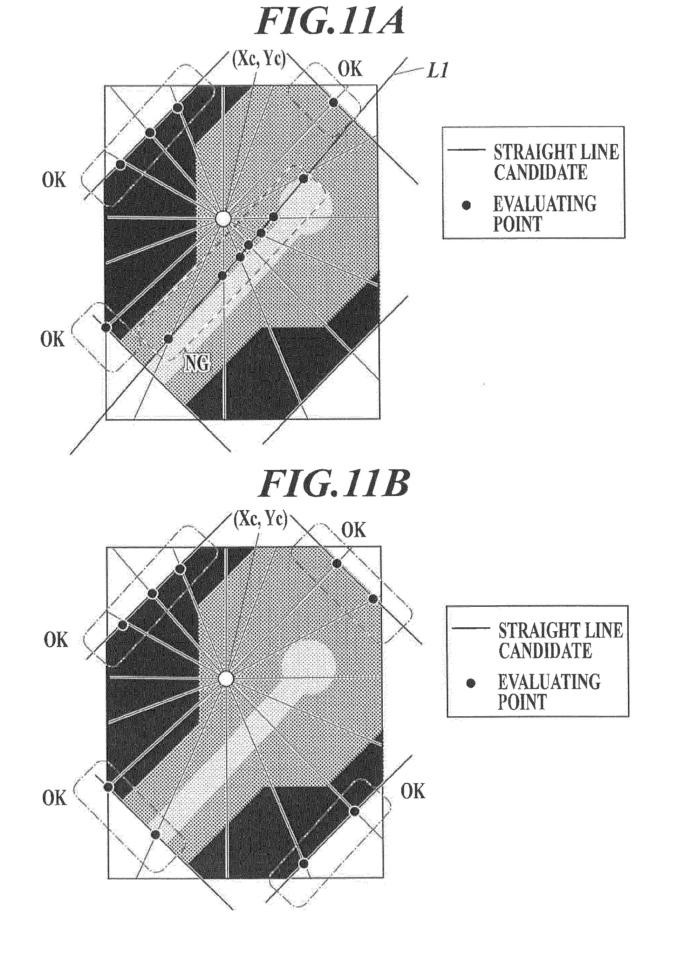

(d1) First, as shown in FIG. 11A, for the straight lines (called searching line here) drawn radially from the reference point (Xc, Yc) in the irradiating field region obtained in step S701 to the edge of the image for every predetermined angle, the control unit 51 sets the point intersecting with the straight line candidate in a position closest to the reference point (Xc, Yc) as the evaluating point (black circle in FIG. 11A).

Next, the control unit 51 calculates the evaluating value of each straight line candidate as the irradiating field edge based on the evaluating point set in each straight line candidate by performing the following processing of (d2) to (d4). Described here is an example showing that as the evaluating value becomes higher, the evaluation as the irradiating field edge becomes lower (evaluation that the feature of not being the irradiating field edge is strong).

(d2) The control unit 51 judges whether each evaluating point is a pass-through point and calculates the percentage of the evaluating points judged to be pass-through on the straight line candidates where the evaluating point is set (first evaluation value). When the percentage of the evaluating point judged to be the pass-through is a predetermined percentage or more, the straight line candidate is judged not to be the irradiating field edge (incorrect) and is removed. Judgment of whether the point is pass-through is performed by the following, for example, when the signal value of the radiation image in the evaluating point substantially matches with the maximum signal value, the evaluating point is judged as the pass-through.

(d3) Next, the control unit 51 calculates the maximum value of the signal value on the outer side (image edge side) than the evaluating points on the straight line candidate (second evaluating value) for each straight line candidate, and when the maximum value is the signal value of pass-through, the straight line candidate is judged to be incorrect and is removed. Pass-through does not occur outside the irradiating field edge, and therefore, the straight line candidate with such evaluating point is removed as not being the irradiating field edge.

(d4) Next, the control unit 51 obtains the inner product of the direction vector of the edge in the evaluating points on the straight line candidate and the normal vector of the straight line candidate for each straight line candidate and calculates the average value (third evaluating value). When the calculated average value is smaller than a predetermined threshold value, the straight line candidate is judged not to be the irradiating field edge (incorrect) and is removed.

Specifically, the inner product of the direction vector of the edge in the evaluating points on the straight line candidate and the normal vector of the straight line candidate can be obtained by the following.

For each straight line candidate, when the formula of the straight line candidate (r= $r_{min}$, θ=$θ_{min}$) with the reference point (Xc, Yc) as the point of origin is the following (formula 16), the normal vector of this straight line candidate is represented by the following (formula 17).

$$rmin = xcosθ_{min} + ysinθ_{min} \ ... \ (formula \ 16)$$

$$(\cos\theta_{min}, \sin\theta_{min}) \dots \text{(formula 17)}$$

When the difference image in the x-direction is Dx (x, y), and the difference image in the y-direction is Dy (x, y), and the coordinates of the evaluating point is (c, l), the direction vector of the edge in the evaluating point (c, l) is the following (formula 18).

$$(Dx (c, l), Dy (c, l)) \dots \text{(formula 18)}$$

With this, the inner product V of the direction vector of the edge in the evaluating point (c, l) and the normal vector of the straight line candidate can be obtained by the following.

$$V = Dx (c, l) \cdot \cos\theta_{min} + Dy (c, l) \cdot \sin\theta_{min} \dots \text{(formula 19)}$$

Here, when the straight line candidate is on the irradiating field edge, the direction vector of the edge on the straight line candidate and the normal vector of the straight line candidate becomes the same direction and the inner product becomes close to 1. However, when the straight line candidate is a structure in a straight line on the human body, the direction vector of the edge does not become the same direction as the normal vector of the straight line candidate. In other words, the inner product becomes smaller than 1. Therefore, when the average value of the inner product of the direction vector of the edge in the evaluating points on the straight line candidate and the normal vector of the straight line candidate is equal to or less than the predetermined threshold value, this is removed from the straight line candidate, and the straight line candidate on the human body can be removed.

According to the above, the direction vector of the straight line candidate can be used instead of the normal vector of the straight line candidate. In this case, when the average value of the inner product of the evaluating point on the straight line candidate is equal to or farther than a predetermined threshold from 0, the straight line candidate is judged not to be the irradiating field edge (incorrect), and is removed.

(d5) The control unit 51 judges the straight line candidate which is not removed in the above described (d1) to (d4) as correct.

The control unit 51 performs the above processing of (d1) to (d5), and when there is a straight line candidate judged to be incorrect, (d1) to (d5) is repeated again in a state with the straight line candidate removed, and when all of the straight line candidates are judged to be correct, the processing of step S704 ends.

For example, in the radiation image shown in FIG. 11A, a straight line L1 is removed in the first processing of (d1) to (d5), and the 4 bold lines shown in FIG. 11B are the straight line candidates. In the next processing of (d1) to (d5), all of the straight line candidates are judged to be correct.

According to the above correct/incorrect judgment method, the searching line is drawn radially from the reference point (Xc, Yc) to the image edge, and the point which intersects with the straight line candidate in a position closest to the reference point (Xc, Yc), is set as the evaluating point. Therefore, when the outline of the irradiating field edge is included in the straight line candidate, the evaluating point is set in the irradiating field region or on the irradiating field edge. In other words, the evaluating value of the correct/incorrect judgment of the straight line candidate is obtained in the irradiating region or on the irradiating field edge. Therefore, the accuracy of judgment of whether the straight line candidate is correct or incorrect can be enhanced.

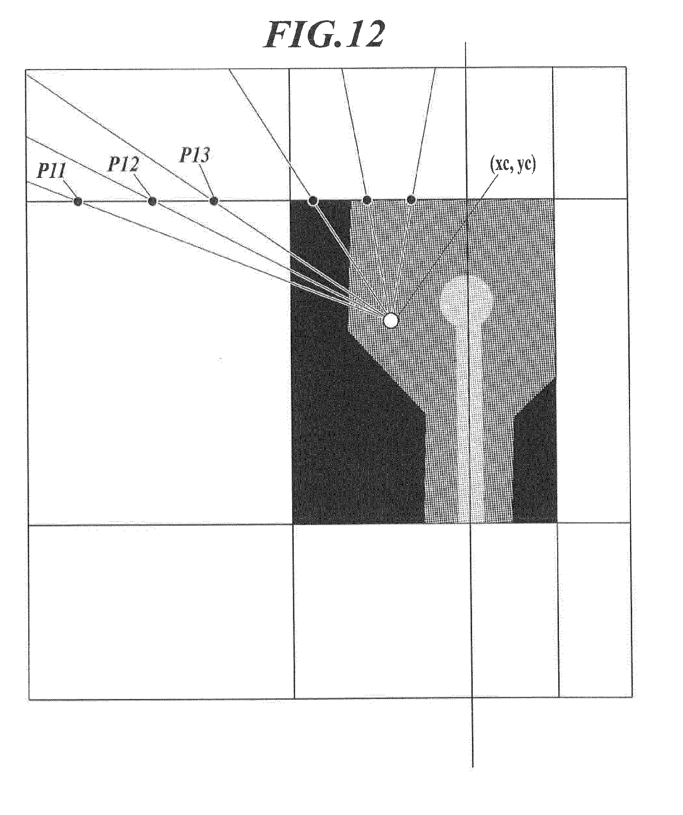

For example, in an image where there is an irradiating field region as shown in FIG. 12, the straight line candidate of the irradiating field edge reaches the region outside the irradiating field. Here, for example, as shown in P11 to P13 of FIG. 12, when an evaluating point is set in the region outside the irradiating field, the direction of the edge is not perpendicular to the straight line candidate (the direction of the edge outside of the irradiating field is in various directions). Therefore, a misjudgment may be made in step (d4). However, according to the method of the present embodiment, when the irradiating field outline is included in the straight line candidate of the irradiating field edge, the evaluating point is always set in the irradiating field region or on the irradiating field edge. Therefore, there is no misjudgment and the correct/incorrect judgment can be performed accurately.

Returning to FIG. 4, the control unit 51 determines the irradiating field region based on the straight line candidate judged to be correct (step S705), and the irradiating field recognition processing ends.

In step S705, the control unit 51 draws a line from the reference point (Xc, Yc) to the image edge. The point where the above line and either of the straight line candidate of the irradiating field edge judged to be correct in step S704 or the image edge line intersect at a position closest to the reference point (Xc, Yc) is to be the irradiating field edge point. This is repeated until going around the reference point (Xc, Yc) once. Then, the region surrounded by the irradiating field edge point is determined as the irradiating field region. The point where the type of straight line where the irradiating field edge point is on changes is determined as the intersecting coordinates of the irradiating field edge line.

When the irradiating field recognition processing ends, the control unit 51 performs the image processing in the irradiating field region recognized in the radiation image (step S8). The image processing performed here is, for example, gradation processing according to subject site, frequency enhancing processing, grain suppressing processing, blackening processing of region outside irradiating field, cutout processing of irradiating field region or the like.

When the image processing ends, the control unit 51 transmits the radiation image with the image processing performed to the server apparatus 10 with the network communication unit 56 (step S9), and the capturing control processing ends.

As described above, the control unit 51 of the capturing console 5 performs the following irradiating field recognizing processing. A reference point is set in the point which is to be the irradiating field region of the radiation image obtained by capturing the subject with radiation using the irradiating field diaphragm. A plurality of irradiating field edge candidate points which are candidates of the point on the irradiating field edge in the radiation image are extracted based on the set reference point. A plurality of straight line candidates of the irradiating field edge are extracted from the radiation image based on the extracted irradiating field edge candidate point. Correct/incorrect judgment is made for each of the extracted straight line candidate, and the irradiating field region in the radiation image is recognized based on the straight line candidate judged to be correct. In the irradiating field recognizing processing, the evaluating value of the straight line candidate as the irradiating field edge is calculated in the range that the straight line candidate is included in the irradiating field region or on the irradiating field edge. The correct/incorrect judgment is performed for each straight line candidate based on the calculated evaluating value. Specifically, a plurality of radial straight lines are set from the reference point to the edge of the radiation image. The intersecting point on the straight line which intersects with the straight line candidate in the position closest to the reference points is set as the evaluating point. The evaluating value of each straight line candidate is calculated based on the evaluating point set on each straight line candidate. With this, the evaluating value of each straight line candidate is calculated in the range that the straight line candidate is included in the irradiating field region or on the irradiating field edge.

Therefore, calculating the evaluating value of the straight line candidate in the region outside the irradiating field prevents misjudgment. With this, the accuracy of judging whether the straight line candidate of the irradiating field edge is correct or incorrect can be enhanced.

Moreover, when the straight line candidate of the irradiating field edge is extracted, the control unit 51 performs the following. Each of the straight lines which pass each extracted irradiating field edge candidate point is represented on the Hough space with the reference point as the point of origin by Hough transform. Polling is performed for the straight line which passes each irradiating field edge candidate point on the Hough space with the polling value weighted based on at least one of edge strength in the irradiating field edge candidate point, signal value of the radiation image between the point that the straight line which passes the irradiating field edge candidate point and the reference point intersects with the image edge and the irradiating field edge candidate point, relation between the edge direction of the irradiating field edge candidate point and the direction of the straight line, and the distance between the irradiating field edge candidate point and the reference point. The straight line candidate is extracted based on the polling result. Therefore, since polling is performed weighting the straight line including features like the irradiating field edge, even the irradiating field edge which is near the image edge and which has few irradiating field edge candidate points can be extracted as the straight line candidate.

Moreover, further to the above polling result, weighting is performed based on the angle between the reference point (90° direction). A predetermined number of straight lines are extracted in order from the large value of the polling result after weighting as the straight line candidate. Therefore, for example, even if the straight line is on the irradiating field edge and the polling value is small because the number of irradiating field edge candidate points are small, etc., the straight line can be extracted as the straight line candidate.

Moreover, when the irradiating field edge candidate point is extracted, the control unit 51 performs the following. The edge strength image showing the edge strength in each pixel of the radiation image is generated. A plurality of radial straight lines are set from the reference point to the edge of the radiation image. The edge strength is calculated for each radial straight line weighted based on the signal value of the radiation image between the points on the above straight line and the point that the straight line intersects with the image edge. The point where the calculated edge strength becomes largest on the same straight line is extracted as the irradiating field edge candidate point. Therefore, the candidate point can be extracted on the irradiating field edge where the edge is weak at the boundary between the human body.

Moreover, when the reference point is set, the control unit 51 generates the image in which the edge strength in each pixel of the radiation image is weighted with the signal value of the radiation image, and the centroid position of the generated image is set as the reference point. Therefore, the reference point can be set in the irradiating field region accurately wherever the irradiating field region is in the image and even when the radiation amount outside the irradiating field is high.

The above description of the present embodiment is one suitable example of the radiation image capturing system of the present invention, and the present invention is not limited to the above.

For example, each of the plurality of steps of the irradiating field recognizing processing described in the above embodiment (step S701 to step S705) can perform highly accurate processing in each step. Therefore, it is preferable that all steps are performed as described above. However, since each of the above are independent processing, some of the steps can be performed differently. For example, the reference point is to be set in the irradiating field region, and the reference point can be set by input on the input unit 53 from the radiation image displayed on the display unit 54. Then, the reference point set as described here can be used in the processing from step S702 and after. Alternatively, for example, the irradiating field edge candidate point, etc. can be extracted by other methods using the reference point set by the method as described in the present embodiment. Alternatively, after performing processing according to the steps S701 to S703 of the present embodiment, the correct/incorrect judgment of the straight line candidate can be performed by other methods.

For example, the above described description discloses an example using a hard disk drive, a nonvolatile memory, etc. as a computer readable medium storing the program which performs the various processing, however, the present invention is not limited to the above example. As other computer readable media, it is possible to apply a portable storage medium such as a CD-ROM, etc. Moreover, as the medium providing data of the program through the communication line, a carrier wave can be applied.

Other than the above, the detailed configuration and the detailed operation of each apparatus composing the radiation image capturing system can be suitably modified without leaving the scope of the present invention.

The present U.S. patent application claims priority under the Paris Convention of Japanese Patent Application No. 2013-268216 filed on December 26, 2013 the entirety of which is incorporated herein by reference.

**Claims**

1. An image processing apparatus comprising:

   a setting unit which sets a point in an irradiating field region of a radiation image obtained by capturing a subject with radiation using an irradiating field diaphragm as a reference point;
   a candidate point extracting unit which extracts a plurality of irradiating field edge candidate points which are candidates of points on an irradiating field edge in the radiation image based on the set reference point;
   a straight line candidate extracting unit which extracts a plurality of straight line candidates of the irradiating field edge from the radiation image based on the extracted irradiating field edge candidate points;
   a judging unit which performs correct/incorrect judgment of whether the extracted straight line candidates are correct or incorrect; and
   a recognizing unit which recognizes the irradiating field region in the radiation image based on the straight line candidates judged to be correct by the judging unit,
   wherein, the judging unit calculates an evaluating value of the straight line candidates as the irradiating field edge within a range where the straight line candidates are included in the irradiating field region or on the irradiating field edge, and performs correct/incorrect judgment of the straight line candidates based on the calculated evaluating value.

2. The image processing apparatus of claim 1, wherein,
   the judging unit sets a plurality of radial straight lines from the reference point toward an edge of the radiation image, sets a point on each straight line where the straight line intersects with the straight line candidate at a position closest to the reference point as an evaluating point, calculates the evaluating value of the straight line candidates based on the evaluating point set on the straight line candidates, and calculates the evaluating value of the straight line candidates within the range where the straight line candidates are included in the irradiating field region or on the irradiating field edge.

3. The image processing apparatus of claim 1, wherein the evaluating value includes an evaluating value calculated based on a normal vector or a direction vector of the straight line candidates and an edge direction of the radiation image at the evaluating point.

4. The image processing apparatus of claim 1, wherein the straight line candidate extracting unit performs the following,

(i) illustrates each straight line which passes the extracted irradiating field edge candidate points on a Hough space with the reference point as a point of origin by Hough transform;

(ii) for the straight line which passes the irradiating field edge candidate points on the Hough space, performs polling with a polling value weighted based on at least one among, edge strength in the irradiating field edge candidate points, signal value of the radiation image between a point that a straight line which passes through the irradiating field edge candidate points and the reference point intersects with an image edge and the irradiating field edge candidate points, relation between an edge direction of the irradiating field edge candidate points and a direction of the straight line, and a distance between the irradiating field edge candidate points and the reference point; and

(iii) extracts the straight line candidate based on the polling result.

5. The image processing apparatus of claim 4, wherein the straight line candidate extracting unit performs weighting on the polling result based on the angle between the reference point, and extracts a predetermined number of straight lines in order from a large value of the polling result after the weighting as the straight line candidates.

6. The image processing apparatus of claim 1, further comprising,

a generating unit which generates an edge strength image showing an edge strength in each pixel of the radiation image,

wherein, the candidate point extracting unit performs the following,

(i) sets a plurality of radial straight lines from the reference point toward an edge of the radiation image;

(ii) for the radial straight lines, calculates an edge strength weighted based on the signal value of the radiation image between points on the straight lines and a point where the straight line intersects with an image edge; and

(iii) extracts the irradiating field edge candidate points where the calculated edge strength becomes maximum on the same straight line.

7. The image processing apparatus of claim 1, wherein the setting unit generates an image in which the edge strength of each pixel of the radiation image is weighted with the signal value of the radiation image, and sets the centroid position of the generated image as the reference point.

8. An irradiating field recognition method comprising:

setting a point in an irradiating field region of a radiation image obtained by capturing a subject with radiation using an irradiating field diaphragm as a reference point;

extracting an irradiating field edge candidate point which is a candidate of a point on an irradiating field edge in the radiation image based on the set reference point;

extracting a straight line candidate of the irradiating field edge from the radiation image based on the extracted irradiating field edge candidate point;

judging to perform correct/incorrect judgment of whether the extracted straight line candidate is correct or incorrect; and

recognizing the irradiating field region in the radiation image based on the straight line candidate judged to be correct,

wherein, in the judging, an evaluating value of the straight line candidate as the irradiating field edge is calculated within a range where the straight line candidate is included in the irradiating field region or on the irradiating field edge, and correct/incorrect judgment of the straight line candidate is performed based on the calculated evaluating value.

# FIG.1

# *FIG.2*

5

CONTROL UNIT — 51

52 — STORAGE UNIT

53 — INPUT UNIT

54 — DISPLAY UNIT

COMMUNICATION I/F — 55

NETWORK COMMUNICATION UNIT — 56

57

# *FIG.3*

```
             ┌─────────────────────────────────┐
             │  CAPTURING CONTROL PROCESSING   │
             └─────────────────────────────────┘
                           │
             ┌─────────────────────────────────┐
             │ SPECIFY CAPTURING ORDER INFORMATION │  S1
             └─────────────────────────────────┘
                           │
             ┌─────────────────────────────────┐
             │   START FPD AND RADIATION SOURCE │
             │       AND ADJUST POSITION         │  S2
             │ AND DIRECTION OF RADIATION SOURCE │
             └─────────────────────────────────┘
                           │
             ┌─────────────────────────────────┐
             │ SET RADIATION IRRADIATING CONDITION │  S3
             │    AND IMAGE READING CONDITION    │
             └─────────────────────────────────┘
                           │
```

NO ◇ IS THERE RADIATION IRRADIATING INSTRUCTION? S4

YES

PERFORM CAPTURING S5

PERFORM CORRECTION PROCESSING S6

PERFORM IRRADIATING FIELD RECOGNITION PROCESSING S7

PERFORM IMAGE PROCESSING S8

TRANSMIT RADIATION IMAGE TO SERVER APPARATUS S9

END

## FIG.4

```
┌─────────────────────────────────┐
│      IRRADIATING FIELD           │
│   RECOGNITION PROCESSING         │
└─────────────────────────────────┘
                │
                ▼
┌─────────────────────────────────┐
│ SET POINT IN IRRADIATING FIELD REGION │  S701
│        AS REFERENCE POINT        │
└─────────────────────────────────┘
                │
                ▼
┌─────────────────────────────────┐
│   EXTRACT IRRADIATING FIELD EDGE │  S702
│        CANDIDATE POINT           │
└─────────────────────────────────┘
                │
                ▼
┌─────────────────────────────────┐
│  EXTRACT STRAIGHT LINE CANDIDATE OF │  S703
│     IRRADIATING FIELD EDGE       │
└─────────────────────────────────┘
                │
                ▼
┌─────────────────────────────────┐
│   JUDGE CORRECT/INCORRECT OF     │
│ EXTRACTED STRAIGHT LINE CANDIDATE │  S704
│    AND REMOVE STRAIGHT LINE      │
│     JUDGED TO BE INCORRECT       │
└─────────────────────────────────┘
                │
                ▼
┌─────────────────────────────────┐
│ DETERMINE IRRADIATING FIELD REGION │  S705
└─────────────────────────────────┘
                │
                ▼
          ┌───────────┐
          │    END    │
          └───────────┘
```

20

# FIG.5

# FIG.6

## FIG.7A

## FIG.7B

# *FIG.8A*

# *FIG.8B*

# *FIG.9*

# FIG.10A

# FIG.10B

# FIG.11A

# FIG.11B

# *FIG.12*

**EP 2 889 836 A2**

## REFERENCES CITED IN THE DESCRIPTION

**Patent documents cited in the description**

- JP 2000023952 A
- JP 3923131 B
- JP 4280729 B
- JP 2013268216 A